## Europäisches Patentamt

## European Patent Office

## Office européen des brevets

(11) Veröffentlichungsnummer: **0 359 972 B1**

(12) # EUROPÄISCHE PATENTSCHRIFT

(45) Veröffentlichungstag der Patentschrift: **09.11.94**

(51) Int. Cl.5: **A61B 5/026**

(21) Anmeldenummer: **89114670.6**

(22) Anmeldetag: **08.08.89**

(54) **Vorrichtung zur optoelektronischen nichtinvasiven Erfassung der Durchflussparameter in menschlichen Extremitäten.**

(30) Priorität: **12.08.88 DE 3827501**

(43) Veröffentlichungstag der Anmeldung:
**28.03.90 Patentblatt 90/13**

(45) Bekanntmachung des Hinweises auf die
Patenterteilung:
**09.11.94 Patentblatt 94/45**

(84) Benannte Vertragsstaaten:
**AT BE CH DE ES FR GB GR IT LI LU NL SE**

(56) Entgegenhaltungen:
**EP-A- 0 282 210**
**EP-A- 0 286 142**
**DE-A- 3 100 610**
**DE-A- 3 146 063**
**DE-A- 3 318 746**

(73) Patentinhaber: **A. Nattermann & Cie. GmbH**
**Nattermannallee 1**
**D-50829 Köln (DE)**

(72) Erfinder: **Schmitt, Hans J., Dr.rer.nat.**
**Hangstrasse 34**
**D-5100 Aachen (DE)**
Erfinder: **Blazek, Vladimir, Dr.**
**Königshügel 31**
**D-5100 Aachen (DE)**

(74) Vertreter: **Münich, Wilhelm, Dr. et al**
**Kanzlei Münich, Steinmann, Schiller**
**Wilhelm-Mayr-Str. 11**
**D-80689 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf eine Vorrichtung zur nichtinvasiven optoelektronischen Erfassung der Durchflußparameter in menschlichen Extremitäten gemäß des Oberbegriffs des Patentanspruchs 1.

Vorrichtungen gemäß dem Oberbegriff des Patentanspruchs 1 sind bspw. aus dem deutschen Offenlegungsschriften 31 00 610, 33 18 746, 36 09 073, 36 09 075 bekannt. Diese bekannten Vorrichtungen ermöglichen eine Erfassung von Blutvolumen- oder Blutdruckschwankungen in menschlichen Extremitäten. Alle diese bekannten Geräte weisen jedoch folgende Nachteile auf:
- Es können keine Blutfluß-Änderungen registriert werden.
- Es können keine mehrkanaligen Untersuchungen gleichzeitig (z.B. in verschiedenen Beinetagen) vorgenommen werden.

Der Erfindung liegt die Aufgabe zugrunde, eine Vorrichtung gemäß dem Oberbegriff des Patentanspruchs 1 derart weiterzubilden, daß Blutfluß-Änderungen erfaßt werden können.

Eine erfindungsgemäße Lösung der gestellten Aufgabe ist mit ihren Weiterbildungen in den Patentansprüchen gekennzeichnet.

Die Erfindung geht von dem Grundgedanken aus, durch getrenntes Normieren der sog. Bewegungs- und Auffüllphase der PPG (photoplethysmographischen)- Kurven mit anschließender Differenzierung der normierten PPG-Kurven oder umgekehrte Vorgehensweise sogenannte normierte Blut-Summenfluß-Kurven (sog. NSF-Kurven) für ein oder mehrere Beinareale zu erhalten.

Da durch diese Vorgehensweise die Differenzen zwischen Maximum- und Startwert bzw. zwischen Minimum und Maximalwert der PPG-Kurve "Eins" werden (abgepumptes bzw. eingeflossenes Blutvolumen = 100 %), werden die Flächeninhalte unter der Bewegungs- und Auffüllphase der NSF-Kurve ebenfalls "Eins" (100 %). Die Höhe der NSF-Kurve gibt die Änderung des Blutvolumina pro Zeiteinheit an, d. h., den aktuellen relativen Blut-Summenfluß. Die NF-Kurve beschreibt somit die zeitabhängige Änderung der Summe aller Blutflüsse im Meßareal infolge einer Streßeinwirkung, z. B. eines gezielten Bewegungsmanövers. Sie hat deshalb eine besondere Relevanz bei der Diagnostik der peripheren Gefäßerkrankungen bzw. der peripheren Hämodynamik. Aus dieser Kurve bzw. aus dem Vergleich verschiedener, gleichzeitig aufgenommener Kurven lassen sich rechnerunterstützt neuartige, aussagefähige Bewertungsparameter für die erwähnte Zielsetzung ermitteln, wie z. B. maximaler Abfluß des Blutes herzwärts während der Bewegungsphase oder maximaler Zufluß des Blutes in die Extremität während der Auffüllphase.

Die Erfindung wird nachstehend anhand eines Ausführungsbeispiels unter Bezugnahme auf folgende Zeichnung näher beschrieben:

Fig. 1    ein Blockschaltbild der erfindungsgemäßen Meßeinrichtung

Fig. 2    eine photoplethysmographische Kurve nach dem Stand der Technik (Blutvolumenänderung infolge einer Beinübung), gemessen im Bereich ME der unteren Extremität

Fig. 3    eine mit der erfindungsgemäßen Vorrichtung generierte Meßkurve, die sog. normierte Blut-Summenfluß-Kurve, gemessen im gleichen Bereich der unteren Extremität wie in Fig. 2 gezeigt

Fig. 4    veranschaulicht die hard- und softwaremäßige Gewinnung der NSF-Kurve, dargestellt am Beispiel eines Patienten mit normaler Hämodynamik im linken Bein und einer phatologischen Hämodynamik im rechten Bein

Fig. 5    ein Computer-Diagnose-Protokoll eines Gefäßgesunden

In sämtlichen Figuren bedeuten:

| | |
|---|---|
| 1 | Rechner |
| 2 | Zentrale Mikroprozessor-Einheit |
| 3 | Einschub-Platine |
| 4a, 4b, 4c, 4d | Treiberstufen für die Optroden |
| 5a, 5b, 5c, 5d | Optroden (Optoelektronische Sensoren) |
| 6 | Multiplex-Schaltkreise |
| 7 | A/D-Wandler |
| 8 | Filter |
| 9 | Normierer |
| 10 | Differentierer |
| UE | Untere Extremität |
| ME | Meßebene |
| I | Bewegungsphase |
| II | Auffüllphase |

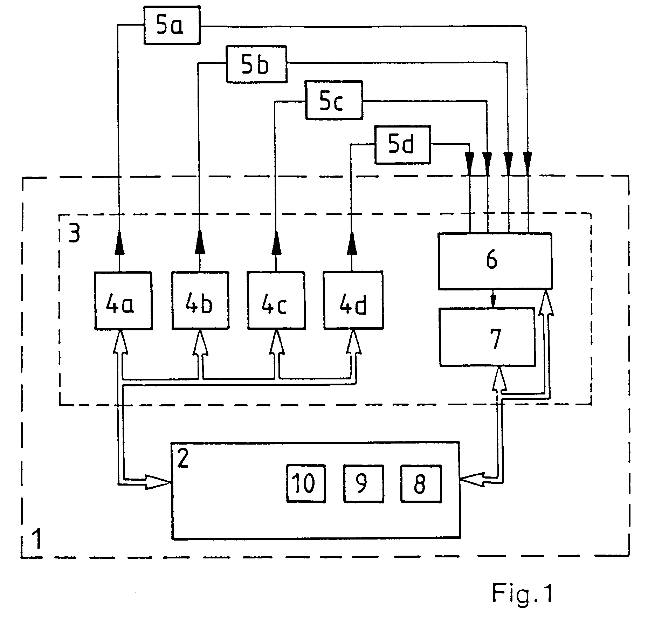

Fig. 1 zeigt schematisch ein vierkanaliges Ausführungsbeispiel der erfindungsgemäßen Meßeinrichtung. Die zentrale Mikroprozessor-Einheit 2 eines handelsüblichen Rechners 1 steuert über ein Datenbus-System spezielle elektronische Schaltkreise 4, 6 und 7, die vorzugsweise auf einer Einschubplatine 3 integriert sind.

Die vier Optroden 5a - 5d beinhalten mindestens je eine Strahlungsquelle und einen Strahlungsdetektor. Sie werden in gewünschten Meßarealen vorzugsweise mit doppelklebenden Folienringen befestigt. In der Regel wird jede Optrode mit der zugehörigen Treiberstufe bzw. mit der Auswerteeinheit elektrisch verbunden; sie beinhaltet dann vorzugsweise auch einen Signalverstärker. Für besondere Anwendungen, wie beispielsweise Untersuchungen der Beinvenenhämodynamik unter Wasser (externe Kompression als Streßmanöver), werden die Optroden mit Lichtwellenleitern mit der Steuer- und Auswerteelektronik verbunden. Nach der Befestigung der Optroden auf der Haut wird zunächst eine Eichphase gestartet. Mikroprozessorgesteuert werden von den Treiberstufen 4a bis 4d so lange individuelle Sendeströme der Optroden eingestellt, bis am Eingang des Multiplexers 6 alle Kanäle brauchbare Signale (ausreichende Signal/Rauschabstand) liefern. Damit fließen durch die Optoden möglichst minimale Ströme, was wiederum die Verluste bzw. die Erwärmung der Optroden minimiert. Für andere diagnostische Fragestellungen (z. B. gezielte Temperaturänderung der Optroden als Streßmanöver) können aber auch gewünschte Optroden-Ströme über den softwaremäßigen Zugriff des Bedieners in die Treiberstufen eingestellt werden. Mit Hilfe der Multiplexer-Schaltung 6 werden die Biodaten tragenden Signale der einzelnen Kanäle vorzugsweise nacheinander geschaltet und anschließend in passendem Format und mikroprozessor-kontrolliert mit A/D-Wandler 7 digitalisiert.

In der zentralen Mikroprozessoreinheit 2 werden schließlich die Signale softwaremäßig auf vorprogrammierte Kriterien überprüft. Werden diese Kriterien (z. B. konstante Beindurchblutung) von allen Kanälen erfüllt, wird vom Rechner automatisch die eigentliche Meßphase gestartet. Gegebenenfalls wird die weitere Messung nicht freigegeben und die Gründe dazu als Fehlermeldung angezeigt. Im folgenden wird die Anwendung der erfindungsgemäßen Vorrichtung am Beispiel der Untersuchung der Beinvenenhämodynamik erörtert. Der Ablauf der Untersuchung besteht aus einer Eichphase, eine Meßphase und einer Analysephase. Die Meßphase wird unterteilt in die Bewegungsphase I und die Auffüllphase II.

In der Eichphase werden nach der Befestigung der Optroden an den Extremitäten Tests der einzelnen Komponenten durchgeführt, gewünschte bzw. vorgegebene Signalstärken eingestellt und u. a. die Meßkriterien überprüft.
Die Meßphase wird - wie bereits erwähnt - vorzugsweise von 2 direkt gestartet. Es ist aber auch vorgesehen, daß der Bedienen den Start der Untersuchung selbst über die Tastatur des Rechners einleitet, wenn ihm die Erfüllung der Startkriterien vom System (z. B. akustisch) signalisiert worden ist. Das gleiche gilt auch für die Bestimmung des Endes der Meßphase.

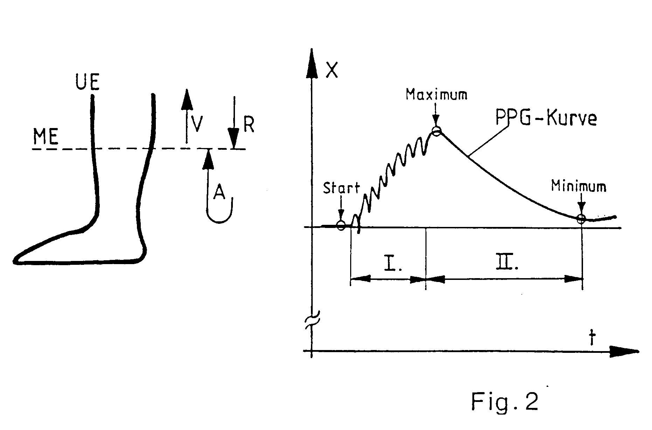

In einem Ruhestand des Patienten herrscht in der Meßebene ME der unteren Extremität UE eine konstante Hautdurchblutung, da der arterielle Bluteinstrom A dem venösen Abfluß V entspricht, wobei der pathologische Reflex R in den Venen gleich Null gesetzt werden kann (s. Fig. 2)

In der Bewegungsphase führt dann der Patient ein vorgegebenes Bewegungsprogramm (z. B. acht Kniebeugen in 16 Sekunden) durch. Infolge der sog. Muskelpumpe wird das venöse Blut vermehrt zum Herzen abtransportiert, so daß in der Meßebene ME gilt V > A + R.

Dadurch sinkt das Blutvolumen im Meßareal, was mit einer Erhöhung der Hautreflexion verbunden ist. Nach Ende der Übung füllt sich in der Phase I das Bein des Venengesunden relativ langsam (nur durch den arteriellen Einstrom A, da V = O, R = O). Beim Venenkranken (R = O) ist die Auffüllphase wesentlich kürzer. Das das optische gewonnene Meßsignal als lokal linear abhängig vom Blutvolumen angenommen werden kann, kann die photopletysmographische (PPG-) Kurve als eine Blutvolumen-Summenkurve interpretiert werden:

- sie steigt im Falle V > A + R,
- sie sinkt im Falle V < A + R.

Die Signalamplitude X der PPG-Kurve kann aufgrund des nicht direkten Meßprinzips nicht geeicht werden, lediglich die Zeitparameter der PPG-Kurve können diagnostisch bewertet werden.

Erfindungsgemäß wird auch dieser Nachteil wie folgt eliminiert:
Die in der zentralen Mikroprozessoreinheit 2 einzeln abgelegten PG-Kurven werden in der Analysephase nun mit 8 gefiltert, mit 9 normiert und schließlich mit 10 differenziert. Die Signalverarbeitungsgruppen 8, 9 und 10 sind vorzugsweise softwaremäßig realisiert; sie können aber auch als hardwaremäßige Schaltkreise aufgebaut werden. Durch die Prozedur werden aus optisch gewonnenen Blutvolumen-Signalen erstmals Blutfluß-Signale abgeleitet.

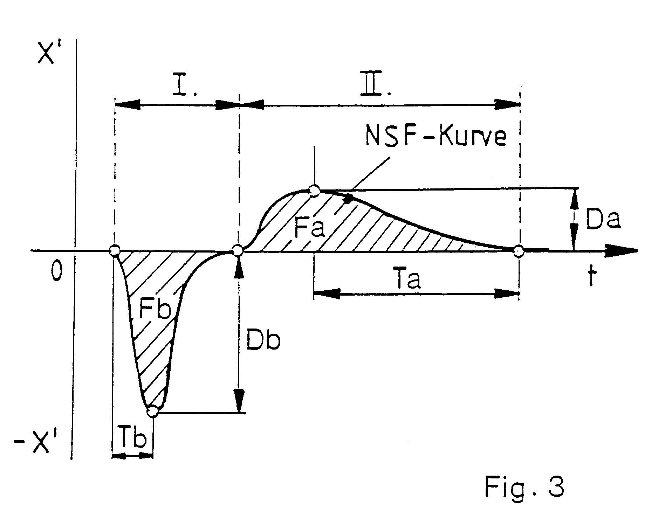

Aus der Blutvolumen-Summenkurve (PPG-Kurve) in Fig. 2 entsteht die sog. normierte Summenfluß-Kurve (NSF-Kurve), (Fig. 3). Unterhalb der Nullinie ist der resultierte Blut-Summenfluß negativ, d. h., das Blut fließt überwiegend herzwärts. Ist die NSF-Kurve positiv, überwiegt ein Blutfluß von zentral nach peripher. Da in einem geschlossenen Blutkreislauf die Summe aller Blutflüsse O ist, gilt Fb = Fa. Flächen unter der NSF-Kurve in der Bewegungs- und Auffüllphase).

Aufgrund der Normierung werden diese Flächen immer gleich 100 % gesetzt. Die Amplitudenparameter Da und Db sind somit neuartige Parameter, die nach dem bisherigen Stand der Technik nicht erreichbar waren. Sie sind beim Venengesunden wesentlich kleiner als beim Venenkranken. Vor allem in Kombination mit den Zeitparametern Ta und Tb (auch diese sind beim Gesunden größer als beim Venenkranken), ermöglichen sie eine wesentlich genauere diagnostische Trennung zwischen normaler und pathologischer Hämodynamik.

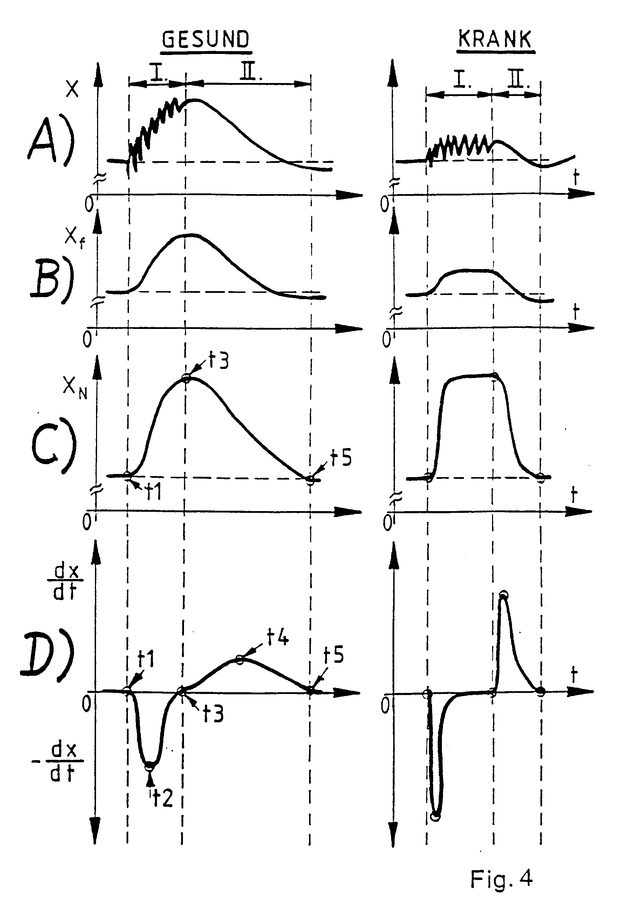

Dieser Sachverhalt wird nochmals veranschaulicht in Fig. 4 am Beispiel eines Patienten mit gesundem linken Bein und krankem rechten Bein.

Die Untersuchung wurde gleichzeitig zweikanalig durchgeführt, die restlichen zwei Kanäle der vierkanaligen Meßeinrichtung wurden zwecks sparen der Speicherplätze softwaremäßig abgeschaltet.

Hier bedeuten:

A) PPG-Kurven (Funktionen X(t))

B) Tiefpaßgefilterte PPG-Kurven Funktionen $X_f(t)$)

C) Normierte und tiefpaßgefilterte PPG-Kurven Funktionen $X_n(t)$)

D) NSF-Kurven $dX_n(t)/dt$

Man erkennt, daß im Vergleich zum gesunden Bein beim venenerkrankten Bein die Parameter Da, Db größer und die Zeitparameter ($t_2 - 1_1$), $t_4 - t_3$) kleiner sind.

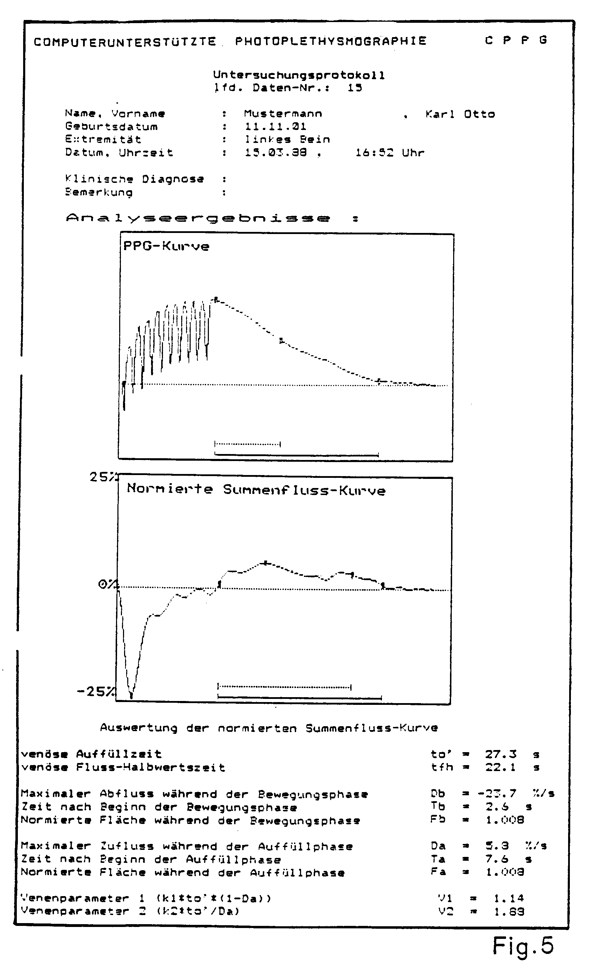

Mit Hilfe von speziellen Softwarepaketen bewertet der Rechner in der der Meßphase folgenden Analysephase die Meßkurven. Als Beispiel zeigt die Fig. 5 eine mögliche Form des Computerprotokolls. Menügesteuert können vom Untersucher aber auch andere Bewertungsprogramme angewählt werden, die z. B. diagnostisch relevante Vergleiche linkes/rechts Bein oder NSF-Kurven in verschiedenen Beinetagen emöglichen.

Mit der beschriebenen Meßvorrichtung können aber auch andere diagnostisch relevante Tests des menschlichen Blutkreislaufs durchgeführt werden, wie z. B.:

- Lagewechsel-Test,
- Okklusions-Test,
- Langzeitanalyse der Blutzirkulations-Rhythmik.

Beim Langzeitwechseltest wird als Streßmanöver die Lagerung des Patienten gezielt geändert. Wird beispielsweise ein Patient mit arterieller Verschlußkrankheit aus einer Beinhochlagerung in eine Beintieflagerung gebracht, setzt im Vergleich zu arteriell Gesunden die arterielle Blutfüllung des Beines bzw. das Sinken der Hautreflexion wesentlich später ein, da der arterielle Einstrom A zu klein ist.

Beim Okklusionstest befindet sich der Patient in einer Ruhelage. Als Streßmanöver dient jetzt eine Sperre entweder des arteriellen Einstroms in die Extremität oder des venösen Abstroms aus der Extremität. Aus der NSF-Kurve während und nach dem Okklusionsmanöver lassen sich rechnerunterstützt sehr interessante Blutfluß- bzw. Kreislaufparameter ermitteln.

Bei der Langzeitanalyse der Blutzirkulations-Rhythmik entfällt das Streßmanöver. Hier werden die endogenen autoregulatorischen Kreislauf-Regelmechanismen beim ruhenden Patienten untersucht, vorzugsweise ebenfalls an mehreren Körperstellen gleichzeitig. Die einzelnen Testabläufe, die Untersuchungsdauer und diverse meßtechnische Parameter (Signalverstärkung, Altersfragment usw.) können vom Benutzer menügesteuert ausgewählt werden. Die dazu benötigten speziellen Dienstprogramme stehen dafür im Speichermedium (Eprom, Magnetplatte) des Rechners zur Verfügung und werden von der Mikroprozessoreinheit 2 kontrolliert geladen.

**Patentansprüche**

1. Vorrichtung (1) zur nichtinvasiven optoelektronischen Erfassung der Durchflußparameter in menschlichen Extremitäten, mit mindestens einer Lichtemissions- und einer Lichterfassungseinrichtung, (5a-d) die zu einem Sensor (Optrode) zusammengefaßt auf die Hautoberfläche aufsetzbar sind, sowie einer Steuer- und Auswerteeinheit (2), die die Lichtemissionseinrichtung steuert und an die das Ausgangssignal der Lichtempfangseinrichtung, das zum Blutvolumen proportional ist, angelegt ist, wobei das Ausgangssignal der Lichtempfangseinrichtung zur Bildung einer Blutvolumen-Summenkurve (PPG-Kurve) wahrend einer Blutentleerung und auffullung der Venen gemessen wird, dadurch **gekennzeichnet**, daß die Steuer- und Auswerteeinheit die Blutvolumen-Summenkurve tiefpaßfiltert (8), normiert (9) und zur Bildung eines dem Blut-Summenfluß (NSF-Kurve) proportionalen Signals differenziert (10).

2. Vorrichtung nach Anspruch 1, dadurch **gekennzeichnet**, daß eine Mehrzahl von Sensoren (Optroden) vorgesehen sind, die an unterschiedlichen Stellen der zu untersuchenden menschlichen Extremität anbringbar sind.

3. Vorrichtung nach Anspruch 1 oder 2, dadurch **gekennzeichnet**, daß die Steuer- und Auswerteeinheit aus den normierten und differenzierten Signal ermittelt, wann der Blut-Summenfluß Null ist, und zu diesem Zeitpunkt die Untersuchung startet.

4. Vorrichtung nach einem der Ansprüche 1 bis 3, dadurch **gekennzeichnet**, daß die Steuer- und Auswerteeinheit aus dem normierten und differenzierten Signal eine Meßendebedingung ermittelt und beim Eintreten der Meßendebedingung die Untersuchung beendet.

5. Vorrichtung nach einem der Ansprüche 1 bis 4, dadurch **gekennzeichnet**, daß die Ausgangssignale der Optroden in einem zugeordneten Zeitfenster digitalisiert und vor der weiteren Auswertung tiefpaßgefiltert werden.

**6.** Vorrichtung nach einem der Ansprüche 1 bis 5,
dadurch **gekennzeichnet**, daß die Steuer- und Auswerteeinheit einen Multiplexer aufweist, der die Ausgangssignale der einzelnen Optroden nacheinander erfaßt.

**7.** Vorrichtung nach einem der Ansprüche 1 bis 6,
dadurch **gekennzeichnet**, daß die Steuer- und Auswerteeinheit Treiberstufen zur Einstellung des Strahlungspegels der Optroden aufweist.

**8.** Vorrichtung nach Anspruch 7,
dadurch **gekennzeichnet**, daß die Steuer- und Auswerteeinheit vor dem Beginn der Messung die Sendeleistung der einzelnen Lichtemissionseinrichtungen so weit erhöht, bis sämtliche Optroden-Ausgangssignale ein bestimmtes Signal/Rauschverhältnis erreichen.

**9.** Vorrichtung nach einem der Ansprüche 1 bis 8,
dadurch **gekennzeichnet**, daß die Lichtemissionseinrichtungen und die Lichtempfangseinrichtungen beabstandet von dem Optroden angeordnet und mit diesen über Lichtwellenleiter verbunden sind.

**10.** Vorrichtung nach einem der Ansprüche 1 bis 9,
dadurch **gekennzeichnet**, daß die Steuer- und Auswerteeinheit einen Mikroprozessor aufweist, der die einzelnen Signale filtert, normiert und differenziert.

**11.** Vorrichtung nach einem der Ansprüche 1 bis 10,
dadurch **gekennzeichnet**, daß der Beginn und das Ende der Meßphase akustisch und/oder optisch angezeigt werden.

**12.** Vorrichtung nach einem der Ansprüche 2 bis 11,
dadurch **gekennzeichnet**, daß die Steuer- und Auswerteeinheit für jede angeschlossene Optrode einzeln diagnostisch relevante Bewertungsparameter errechnet, die Signale der einzelnen Kanäle vergleicht und deren Unterschied anzeigt.

**Claims**

**1.** Device (1) for the non-invasive opto-electronic detection of the flow parameters in human extremities, comprising at least one light emitter and at least one light receiver means (5a - d) adapted to be combined to form a single sensor (optrode) for being placed onto the surface of the skin, as well as one control and evaluation means (2) which controls said light emitter means and to which the output signal from said light receiver means is applied, which output is proportional to the blood volume, with the output signal from said light receiver means being measured for forming a blood volume summation curve (PPG curve) during one blood discharge and supply cycle of the veins,
**characterized** in that said control and evaluation means subjects said blood volume summation curve (8) to a low-pass filtering step (8), normalizes (9) and differentiates (10) it so as to form a signal proportional to the cumulative blood flow (NSF curve).

**2.** Device according to Claim 1,
**characterized** in that a plurality of sensors (optrodes) is provided which are adapted to be placed at different sites on the human extremity to be examined.

**3.** Device according to Claim 1 or 2,
**characterized** in that said control and evaluation means establishes from the normalized and differentiated signal when the cumulative blood flow is zero, and starts the examination cycle by that point of time.

**4.** Device according to any of Claims 1 to 3,
**characterized** in that said control and evaluation means establishes a measurement termination condition from said normalized and differentiated signal, and terminates the examination cycle upon occurrence of the measurement termination condition.

**5.** Device according to any of Claims 1 to 4,
**characterized** in that the output signals from said optrodes are digitized in an associated time window and are subjected to a low-pass filtering operation prior to their further evaluation.

**6.** Device according to any of Claims 1 to 5,
**characterized** in that said control and evaluation means comprises a multiplexer which detects the output signals from the individual optrodes successively.

**7.** Device according to any of Claims 1 to 6,
**characterized** in that said control and evaluation means comprises driver stages for setting the radiation level of said optrodes.

8. Device according to Claim 7,
**characterized** in that said control and evaluation means increases the emitted output of the individual light emitter means prior to the beginning of a measurement cycle until all of the optrode output signals achieve a specified signal-to-noise ratio.

9. Device according to any of Claims 1 to 8,
**characterized** in that said light emitter means and said light receiver means are spaced from said optrodes and are connected to the latter via light guides.

10. Device according to any of Claims 1 to 9,
**characterized** in that said control and evaluation means comprises a microprocessor which filters, normalizes and differentiates the individual signals.

11. Device according to any of Claims 1 to 10,
**characterized** in that the beginning and the end of the measuring phase are acoustically and/or optically signalled.

12. Device according to any of Claims 1 to 11,
**characterized** in that said control and evaluation means computes diagnostically pertinent rating parameters for each optrode connected, compares the signals of the individual channels, and displays their differences.

**Revendications**

1. Dispositif (1) pour la détermination opto-électronique non-intrusive des paramètres du flux sanguin dans des extrémités humaines, comprenant au moins un moyen à émetteur de lumière et au moins un moyen à récepteur de lumière (5a - d) appropriés à être combinés pour former un seul détecteur (optrode) à placer sur la surface de la peau,
et également un moyen de commande et d'évaluation (2) qui commande ledit moyen à émetteur de lumière, et auquel est appliqué le signal de sortie qui provient dudit moyen à émetteur de lumière, lequel signal de sortie est proportionnel au volume de sang, le signal de sortie du moyen à récepteur de lumière étant mesuré pour produire un tracé cumulatif du volume sanguine (tracé PPG) au cours d'un cycle d'évacuation et amenée de sang dans les veines,
**caractérisé** en ce que ledit moyen de commande et d'évaluation soumet ledit tracé de volume sanguin cumulatif (8) à une opération de filtrage passe-bas (8), le normalise (9) et fait une différenciation (10) pour produire un signal proportionnel au flux sanguin cumulatif (tracé NSF).

2. Dispositif selon la revendication 1,
**caractérisé** en ce qu'une pluralité de détecteurs (optrodes) est prévue, qui sont appropriés à être placés aux sites différentes sur l'extrémité humaine à examiner.

3. Dispositif selon la revendication 1 ou 2,
**caractérisé** en ce que ledit moyen de commande et d'évaluation établit, à la base du signal normalisé et différencié, quand un flux sanguin cumulatif est zéro, et déclenche le cycle d'examen à ce moment.

4. Dispositif selon une quelconque des revendications 1 à 3,
**caractérisé** en ce que ledit moyen de commande et d'évaluation établit une condition de bout de mesure à la base dudit signal normalisé et différencié, et finit le cycle d'examen après l'occurrence de la condition de bout de mesure.

5. Dispositif selon une quelconque des revendications 1 à 4,
**caractérisé** en ce que les signaux de sorties desdites optrodes sont convertis en numérique au dedans d'une fenêtre de temps appartenant, et sont soumis à une opération de filtrage passe-bas avant leur évaluation ultérieure.

6. Dispositif selon une quelconque des revendications 1 à 5,
**caractérisé** en ce que ledit moyen de commande et d'évaluation comprend un multiplexeur qui détecte, en succession, les signaux de sortie des optrodes individuelles.

7. Dispositif selon une quelconque des revendications 1 à 6,
**caractérisé** en ce que ledit moyen de commande et d'évaluation comprend des étages d'attache pour l'ajustement du niveau de rayonnement desdites optrodes.

8. Dispositif selon la revendication 7,
**caractérisé** en ce que ledit moyen de commande et d'évaluation augmente la sortie émise desdits moyens individuels à émetteur de lumière, avant le commencement d'un cycle de mesure, jusqu'à ce que tous les signaux de sortie des optrodes atteignent un rapport signal/bruit spécifié.

9. Dispositif selon une quelconque des revendications 1 à 8,

**caractérisé** en ce que lesdits moyens à émetteur et à récepteur de lumière sont écartés desdites optrodes et sont reliés aux dernières moyennant des guides d'ondes lumineuses.

10. Dispositif selon une quelconque des revendications 1 à 9,
**caractérisé** en ce que ledit moyen de commande et d'évaluation comprend un microprocesseur pour le filtrage, la normalisation et la différenciation des signaux individuels.

11. Dispositif selon une quelconque des revendications 1 à 10,
**caractérisé** en ce que le commencement et le bout du stade de mesure sont signalisés par des moyens acoustiques et/ou optiques.

12. Dispositif selon une quelconque des revendications 1 à 11,
**caractérisé** en ce que ledit moyen de commande et d'évaluation calcule des paramètres correspondants d'évaluation pour chaque optrode y reliée, fait une comparaison entre les signaux des voies individuelles, et affiche les différences entre eux.

Fig.1

Fig. 2

Fig. 3

Fig. 4

COMPUTERUNTERSTÜTZTE PHOTOPLETHYSMOGRAPHIE       C P P G

Untersuchungsprotokoll
lfd. Daten-Nr.:  15

Name, Vorname        :  Mustermann          , Karl Otto
Geburtsdatum         :  11.11.21
Extremität           :  linkes Bein
Datum, Uhrzeit       :  15.03.39 ,    16:52 Uhr

Klinische Diagnose   :
Bemerkung            :

Analyseergebnisse  :

PPG-Kurve

25%

Normierte Summenfluss-Kurve

0%

-25%

Auswertung der normierten Summenfluss-Kurve

| | | | |
|---|---|---|---|
| venöse Auffüllzeit | to' = | 27.3 | s |
| venöse Fluss-Halbwertszeit | tfh = | 22.1 | s |
| Maximaler Abfluss während der Bewegungsphase | Db = | -23.7 | %/s |
| Zeit nach Beginn der Bewegungsphase | Tb = | 2.6 | s |
| Normierte Fläche während der Bewegungsphase | Fb = | 1.008 | |
| Maximaler Zufluss während der Auffüllphase | Da = | 5.3 | %/s |
| Zeit nach Beginn der Auffüllphase | Ta = | 7.6 | s |
| Normierte Fläche während der Auffüllphase | Fa = | 1.003 | |
| Venenparameter 1 (k1∗to'∗(1-Da)) | V1 = | 1.14 | |
| Venenparameter 2 (k2∗to'/Da) | V2 = | 1.69 | |

Fig.5